# EUROPEAN PATENT APPLICATION

(11) **EP 4 382 593 A1**
(43) Date of publication of application: **12.06.2024**
(21) Application number: 22206255.6
(22) Date of filing: 08.11.2022
(51) Int. Cl.: C12M 1/00, C12M 1/02, C12M 1/34

(54) **BIOREACTOR VESSEL**

(71) Applicant: The Automation Partnership (Cambridge) Ltd., Hertfordshire SG8 5WY (GB)
(72) Inventor: SULLIVAN, Sean, Royston, SG8 5WY (GB)
(74) Representative: Mewburn Ellis LLP

(57) **Abstract**

A bioreactor vessel is provided for performing cell cultivation. The vessel has a stirrer within the vessel for stirring, in use, a cell culture solution contained therewithin, and is configured for performance of closed-loop control of cell cultivation process parameters. The vessel has a reserved culture volume (V_{rc}) for the cell culture solution and a reserved headspace volume (Vᵣₕ) for gas above the reserved culture volume, V_{rc} + Vᵣₕ being equal to the total volume (Vₜₒₜ) of the vessel, V_{rc} being less than 15 mL, and the ratio Vᵣₕ/V_{rc} being in the range from 0.5 to 3.

## Description

### Field of the Invention

The present invention relates to a bioreactor vessel for performing cell cultivation.

### Background

Cell cultivation is the performance of cell growth or fermentation in a favourable artificial environment. Example applications of cell cultivation are for the production of mAbs, vaccines, or recombinant proteins, and cell and gene therapies.

Cell line selection and early process development for cell cultivation is often performed using parallel cultivations in small scale, benchtop bioreactor vessels, such as the Ambr 15^{™} microbioreactor system from Sartorius AG which enables 24 or 48 parallel cultivations with disposable bioreactor vessels holding volumes of cell culture solution in the range from 10 to 15 mL. EP A 2270129 (the entirety of which is incorporated herein by reference) describes a cell culture module for a similar microbioreactor system, the module comprising: a base including a receiving station for removably receiving a plurality of bioreactor vessels at respective locations; a drive mechanism; and a clamp plate, removably connectable to the base and connectable to the drive mechanism, for transmitting input motion from the drive mechanism into multiple rotary motion outputs, each output corresponding to a respective vessel location in the receiving station.

Promising cell lines and processes identified from these microbioreactor systems can be further developed by parallel cultivations in higher throughput and higher volume bioreactor vessels, an example being the Ambr 250^{™} bioreactor system from Sartorius AG which performs high throughput cell cultivation with 12 or 24 disposable bioreactors for volumes of cell culture solution in the range from 100 to 250 mL. The best candidates from these developments can ultimately be transferred to larger scale bioreactor vessels such as the Biostat Cplus^{™} sterilizable-in-place bioreactor also from Sartorius which allows working volumes of from 5 L to 30 L.

This streamlined approach to development allows many variables to be explored, while promoting cost-effective experimentation by making efficient use of labour, facility space, capital, media and consumables.

For successful and efficient optimisation of process variables, it is important that the systems enable accurate and reproducible closed-loop control of process parameters, such as stirrer speed, temperature, pH, dissolved oxygen concentration (DO), and gas composition in the headspace of the bioreactor vessels. This control is also important for successful process upscaling on transfer to higher volume bioreactors.

For the smallest scale bioreactors vessels, this control can be particularly difficult to exercise. For example, a small vessel has a lower volume to surface ratio of the cell culture solution within the vessel than a large vessel, which can make uniformity of process conditions throughout the cell culture solution within a small vessel difficult to achieve.

Conventionally, these difficulties have placed a lower limit on the volume of bioreactor vessels for cell cultivation. For example, the bioreactor vessels of the Ambr 15^{™} microbioreactor system each have a reserved culture volume (V_{rc}) for the cell culture solution placed in the vessel of 15 mL, the reserved culture volume being less than the total volume (Vₜₒₜ) of the vessel such that there is also a reserved headspace volume (Vᵣₕ = Vₜₒₜ- V_{rc}) for gas above the reserved culture volume. In use, the volume of cell culture solution placed in the vessel can be less than the reserved culture volume (in which case the actual headspace volume will be greater than the reserved headspace volume), the Ambr 15^{™} microbioreactor system, for example, allowing cell culture solution volumes of down to 10 mL. Indeed, in practice, it is quite usual to start cell cultivation with a relatively small volume of cell culture solution, and then to grow that volume by liquid additions during cultivation (although sampling extractions can also reduce the volume during cultivation). However, under no circumstances is the volume of cell culture solution in the vessel allowed to become greater than the reserved culture volume, as this would lead to the actual headspace volume being less than the reserved headspace volume, which can cause loss of control of the cultivation conditions by loss of control of the gas composition within the headspace.

### Summary of the Invention

Despite the success of conventional microbioreactor systems, it would be desirable to be able to perform cell cultivation for cell line selection and early process development using even smaller volumes of cell culture solution, as this would further promote cost-effective experimentation.

The present invention has been devised in light of the above considerations. In particular, the present inventor has surprisingly found that, despite conventional expectations, it is possible to adequately control process parameters during cell cultivation even when the reserved culture volume (V_{rc}) for the cell culture solution is less than 15 ml.

Accordingly, in a first aspect the present invention provides a bioreactor vessel for performing cell cultivation, the vessel having a stirrer within the vessel for stirring, in use, a cell culture solution contained therewithin, and being configured for performance of closed-loop control of cell cultivation process parameters, wherein the vessel has a reserved culture volume (V_{rc}) for the cell culture solution and a reserved headspace volume (Vᵣₕ) for gas above the reserved culture volume, V_{rc} + Vᵣₕ being equal to the total volume (Vₜₒₜ) of the vessel, V_{rc} being less than 15 mL, and the ratio Vᵣₕ/V_{rc} being in the range from 0.5 to 3.

As noted above, in practice, it is quite usual to start cell cultivation with a relatively small volume of cell culture solution, and then to grow that volume by liquid additions during cultivation, but the volume of cell culture solution in the vessel is not allowed to become greater than the reserved culture volume. In general, a lower ratio of Vᵣₕ/V_{rc} makes gassing via control of the headspace gas mix more difficult to control, while a higher ratio means the total volume (Vₜₒₜ) of the vessel has to be increased. By having Vᵣₕ/V_{rc} in the range from 0.5 to 3, the inventor has found that these opposing interests can be reconciled, whereby the total volume of the vessel is such that the vessel can be miniaturised, while the actual headspace volume in the vessel during any given cell cultivation allows the gas composition within the headspace to be adequately controlled. Moreover, despite having a V_{rc} which is less than 15 mL, allowing actual culture volumes of around 2 or 3 mL, it has been found that successful performance of closed-loop control of process parameters is still achievable.

Preferably, the ratio Vᵣₕ/V_{rc} is in the range from 1 to 3. More preferably, Vᵣₕ/V_{rc} is in the range from 2 to 3. At low culture volumes, sparging as a means of introducing gas is very difficult to control, as it can lead to foaming and cell damage. In the absence of sparging, the importance of being able to accurately control gas composition in the headspace increases. Higher values of Vᵣₕ relative to V_{rc} provides the headspace with more capacity to control the culture conditions, and what may otherwise have to be very small gas additions to the headspace can be avoided. In particular, it can be difficult to make accurate and timely gas additions into a small volume headspace, for example as the volume of the gas delivery system becomes significantly larger than that of the headspace.

The cell cultivation process parameters may typically include stirrer speed and/or culture solution temperature.

Conveniently, the vessel may have a gas inlet port and a gas outlet path for controlling the composition of gas in the headspace above the cell culture solution. In this case, the process parameters may include flow rate of gas into the headspace through the gas inlet port.

The reserved culture volume (V_{rc}) may be 10 mL or less, preferably is 8 mL or less, and more preferably is 6 mL or less.

The vessel may have side walls which are configured to produce, at a predetermined height, a step change in the internal cross-sectional area of the vessel as measured perpendicularly to the height direction, the predetermined height defining a boundary between the reserved culture volume (V_{rc}) and the reserved headspace volume (Vᵣₕ). The step change is typically a step change increase in the internal cross-sectional area above the predetermined height. The side walls may incorporate shoulders to produce the step change.

The vessel may be further configured to have a minimum culture volume (V_{minc}) for cell culture solution in the vessel of 2 mL or more. The minimum culture volume is the smallest volume of cell culture solution that can be placed in the vessel while still enabling cell cultivation with closed-loop control of the process parameters. That is, at lower volumes than 2 mL, it becomes difficult to accurately and reproducibly perform closed-loop control of the process parameters. Like the reserved culture volume and the reserved headspace volume, the minimum culture volume is a property of the vessel. V_{minc}/V_{rc} may be 0.2 or more, and preferably 0.3 or more. V_{minc}/V_{rc} may be 0.6 or less, and preferably 0.4 or less.

The minimum culture volume (V_{minc}) may be determined by a requirement for the stirrer to enter the cell culture solution. For example, the stirrer may in general be an impeller. In this case, the minimum culture volume (V_{minc}) may be determined by a requirement for the cell culture solution to fully cover the impeller.

Additionally, or alternatively, the minimum culture volume (V_{minc}) may be determined by a requirement for the cell culture solution to have a minimum depth. For example, the vessel may have one or more sensing spots on the bottom of the vessel (e.g. pH and/or DO sensing spots). In this case, the minimum culture volume (V_{minc}) may be determined by a requirement for the cell culture solution to cover the one or more sensing spots to a minimum depth. Indeed, more generally, the process parameters may further include pH and/or DO of the culture solution.

In a second aspect the present invention provides a bioreactor system including a cell culture module comprising a base having a receiving station configured to removably receive and controllably interface to a plurality of bioreactor vessels at respective vessel locations, the bioreactor system further including plural of the bioreactor vessels of the first aspect received at and interfaced to the respective vessel locations, and a controller to perform closed-loop control of the process parameters for each bioreactor vessel;
wherein the cell culture module further comprises:
a drive mechanism; and
a transmission connectable to the drive mechanism for transmitting input motion from the drive mechanism into multiple rotary motion outputs, each output being removably connected to a stirrer of a respective bioreactor vessel for rotation of that stirrer.

For example, the transmission can be a clamp plate removably connectable to the base and connectable to the drive mechanism. Such a clamp plate facilitates the set up of the system and improves the efficiency of the turnaround between experiment runs because all that is required is to insert the vessels in position within the receiving station, then to connect the clamp plate to the base and the drive mechanism, which action connects the drive mechanism to all of the vessels. There is therefore no need to make individual connections for every vessel. Conveniently, the clamp plate can contain respective gas lines for removably connecting to gas inlet ports of the bioreactor vessels.

When the process parameters further include DO and/or pH of the culture solution, the respective vessel locations may conveniently have corresponding sensors for interfacing to the bioreactor vessels and measuring DO and/or pH of the culture solution.

In a third aspect the present invention provides a kit of parts including a bioreactor system, and two sets of bioreactor vessels for performing cell cultivation;
wherein each vessel has a stirrer within that vessel for stirring, in use, a cell culture solution contained therewithin, is configured for performance of closed-loop control of cell cultivation process parameters, and has a reserved culture volume (V_{rc}) for the cell culture solution and a reserved headspace volume (Vᵣₕ) for gas above the reserved culture volume, V_{rc} + Vᵣₕ being equal to the total volume (Vₜₒₜ) of that vessel;
wherein the bioreactor vessels are identical to each other within each set, the bioreactor vessels of a first one of the sets are bioreactor vessels according to the first aspect, and the reserved culture volume (V_{rc}) of the vessels of the first one of the sets is less than that of the vessels of the second one of the sets;
wherein the bioreactor system includes a cell culture module comprising a base having a receiving station configured to removably receive and controllably interface to the bioreactor vessels of each of the sets at respective vessel locations, and further includes a controller which, when the bioreactor vessels of one of the sets are received at and interfaced to the respective vessel locations, is configured to perform closed-loop control of the process parameters for each bioreactor vessel of that set; and
wherein the cell culture module further comprises:
   a drive mechanism; and
   a transmission connectable to the drive mechanism for transmitting input motion from the drive mechanism into multiple rotary motion outputs, each output being removably connectable to a stirrer of a respective bioreactor vessel when received at and interfaced to a respective vessel location for rotation of that stirrer.

Advantageously, the bioreactor system of the kit of parts of the third aspect can receive bioreactor vessels having different reserved culture volumes (V_{rc}), which allows promising cell lines and processes identified using the smaller vessels of the first set to be further developed using the larger vessels of the second set in the same bioreactor system.

The reserved culture volume (V_{rc}) of the vessels of the first one of the two sets may be at least half that of the vessels of the second one of the two sets.

The bioreactor vessels of the second one of the sets may also be bioreactor vessels according to the first aspect. Alternatively, they may have a reserved culture volume (V_{rc}) of 15 mL or more, and/or they may have Vᵣₕ/V_{rc} outside the range from 0.5 to 3.

As with second aspect, the transmission can be a clamp plate removably connectable to the base and connectable to the drive mechanism. The clamp plate can contain respective gas lines for removably connecting to gas inlet ports of the bioreactor vessels. Also as with second aspect, the respective vessel locations may have sensors for interfacing to the bioreactor vessels and measuring DO and/or pH of the culture solution.

In a fourth aspect the present invention provides a method of performing cell cultivation including:
providing the bioreactor vessel of the first aspect;
introducing less than 10 mL of cell culture solution into the bioreactor vessel at a start of cell cultivation; and
performing closed-loop control of cell cultivation process parameters to cultivate cells within the introduced cell culture solution.

8 mL or less, preferably 6 mL or less, and more preferably 4 mL or less, of cell culture solution may be introduced into the bioreactor vessel at the start of cell cultivation.

The method may further include introducing additional cell culture solution into the bioreactor vessel after the start of cell cultivation. However, in this case, the total amount of cell culture solution in the bioreactor vessel does not exceed the reserved culture volume (V_{rc}) of the vessel for the cell culture solution.

The invention includes the combination of the aspects and preferred features described except where such a combination is clearly impermissible or expressly avoided.

### Summary of the Figures

Embodiments and experiments illustrating the principles of the invention will now be discussed with reference to the accompanying figures in which:
**Figure 1** is a perspective overview of an automated microscale bioreactor system;
**Figure 2** is a perspective view of a bioreactor vessel for use in the bioreactor system;
**Figure 3** is a perspective view of a cell culture module, which comprises part of the bioreactor system;
**Figure 4** is a perspective view of the cell culture module, with a drive coupling and clamp plate removed;
**Figure 5** is a perspective view of the drive coupling, showing projecting drive pin;
**Figure 6** is a top perspective view of the clamp plate; and
**Figure 7** is a perspective view of another bioreactor vessel for use in the bioreactor system.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

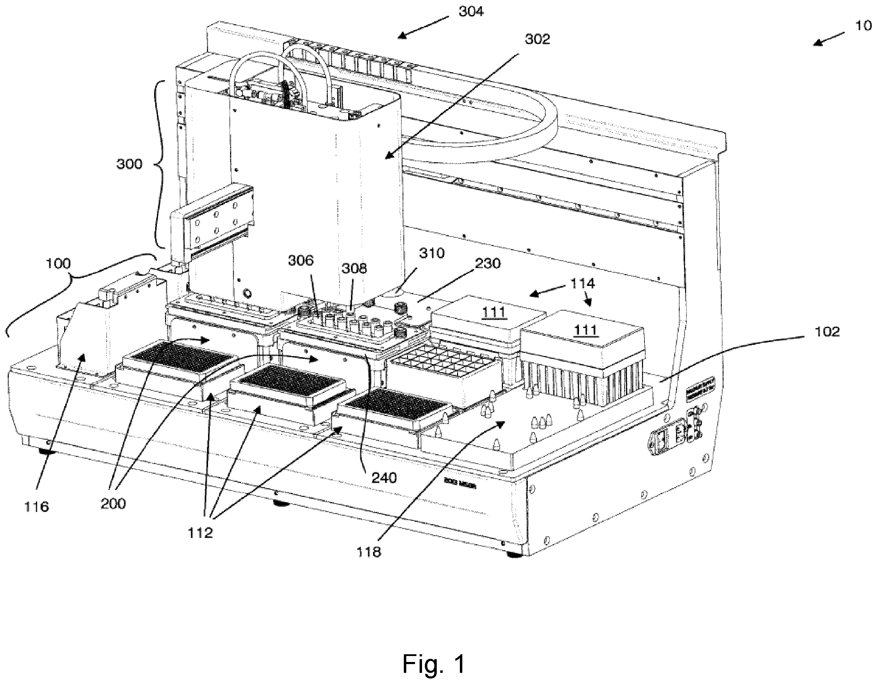

A bioreactor processing system 10 of the type disclosed in EP A 2270129 comprises, generally, a bed station 100 and a liquid handling station 300, which may be interconnected (as shown in Figure 1) or may be separate from one another.

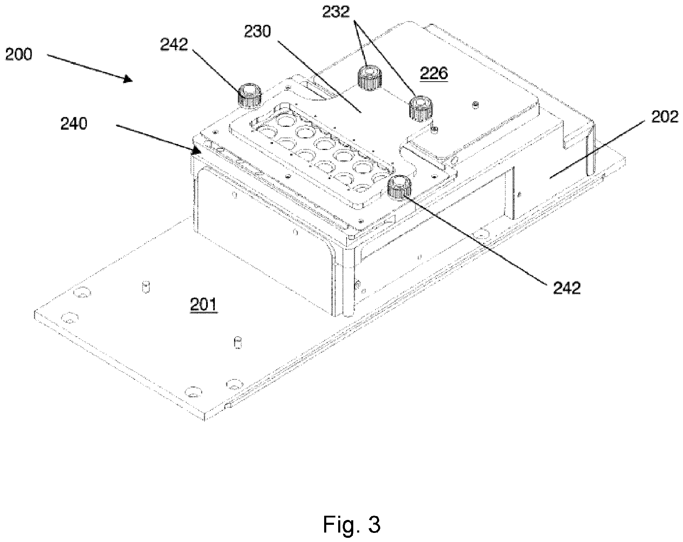

The bed station 100 comprises a frame 102 on which are mounted various modules. The modules include at least one cell culture module 200, which is described in greater detail below with reference to Figs. 3A and 3|B, and, optionally, one or more well plate modules 112, pipette tip box modules 114, pipette tip waste modules 116 and lid storage modules 118. Each module may include a removable lid 111. A well plate module 112 may contain one or more wells, which may be shallow or deep.

The liquid handling station 300 includes a head 302 mounted on a conventional X-Y positioning robot 304. The head 302 includes components that are selectively moveable along the Z axis. The head 302 is thus being capable of addressing and interacting with each of the modules, as will be described in greater detail below.

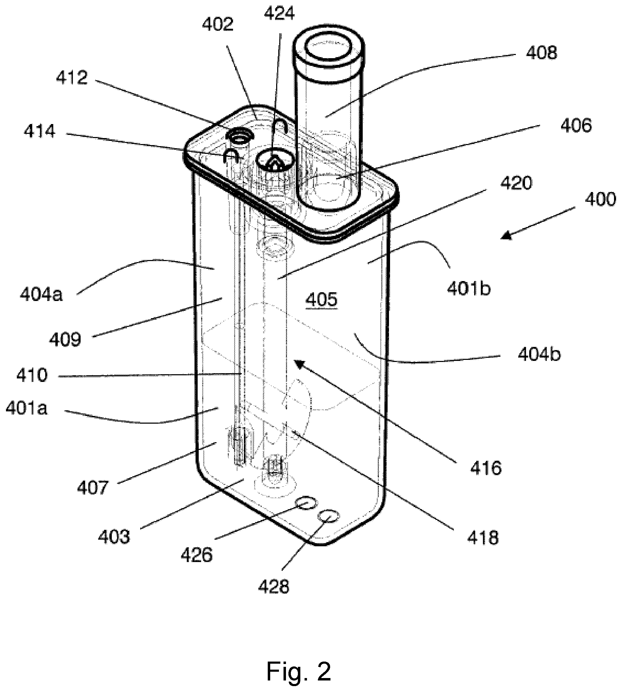

With reference to Figure 2, each vessel 400 of a set of identical microscale bioreactor vessels for use with the bioreactor system 10 comprises front, back, top, bottom and side walls 401a, 401b, 402, 403, 404a, 404b defining a chamber 405 for receiving a cell culture solution 407 having a headspace 409 above. The top wall 402 includes a fluid transfer port 406, on which is removably attached a cap 408. The vessel further includes a sparge tube 410, having a gas input port 412 in the top wall 402 of the vessel. The gas input port 412 includes a filter 414.

A stirrer 416 in the form of an impeller comprises blades 418 mounted at the base of a vertical shaft 420, and is rotatably mounted within the vessel 400. The upper end of the shaft 420 includes a drive input 424. A pH sensor spot 426 and a DO sensor spot 428 are disposed on the bottom wall 403, such that they are able to detect the pH and DO levels of the solution 407 and to be interrogated from the exterior of the vessel 400.

Venting of the vessel chamber 405 is achieved via a labyrinthine path connecting the headspace 409 to atmosphere via the stirrer shaft drive input 424. Alternatively, a separate vent port may be provided towards the top of the vessel 400.

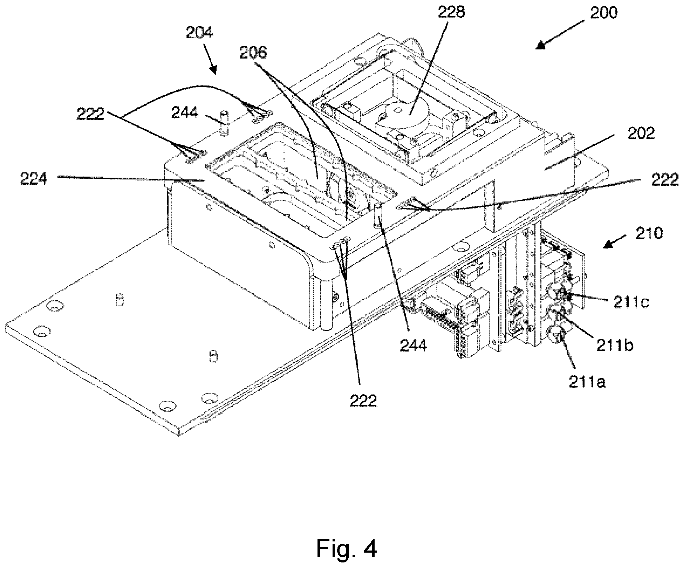

As shown in Figure 1, the bioreactor processing system 10 comprises a pair of cell culture modules 200 mounted to the deck 102 of the base station 100. It will be understood, however, that the system may include just a single such cell culture module 200 or more than two such cell culture modules 200. For the purposes of description, reference is made to Figure 3, showing a single cell culture station 200 in isolation, and Figure 4, which corresponds, but has some parts removed for clarity.

The cell culture module 200 comprises a base 202 mounted on a base plate 201. The base plate 201 is removably connectable to the base station 100. The cell culture module base 202 includes a receiving station 204 for removably receiving a plurality of bioreactor vessels 400. As illustrated, the receiving station 204 can hold up to twelve vessels 400 in two rows of six at respective locations 206. Thus, the bioreactor processing system 10 having a pair of such modules 200 has the capacity to process up to 24 vessels simultaneously. It will be appreciated, however, that the receiving station 204 could be designed to accommodate a greater or lesser number of vessels 400 and that the vessels 400 could be arranged in any suitable configuration.

One or more heaters (not shown) are located adjacent to the vessel receiving locations 206 to control the temperature of the vessels.

A valve assembly 210 is mounted to the underside of the cell culture module base 202. The valve assembly 210 is received in a cavity of the bed station 100 when the cell culture module 200 is connected to the bed station. The valve assembly 210 has three input ports 211a-c, respectively connectable to O₂, N₂ and CO₂ gas supplies. A bank of valves is associated with each respective input port 211a-c, each bank comprising a valve for each vessel receiving location 206. Thus, in the illustrated embodiment, the valve assembly comprises a total of 36 valves 214. From another point of view, each vessel receiving location 206 has three associated valves: one to control the supply of O₂, another to control the supply of N₂ and another to control the supply of CO₂. Further discussion of the valve assembly 210 can be found in EP A 2270129.

The cell culture module base 202 further includes a drive mechanism, indicated generally at 226. The drive mechanism includes a motor (not shown) connected to a parallelogram linkage 228 to produce an eccentric output motion. A drive coupling 230 is connected, via thumbscrews 232, to the parallelogram linkage and is hence driven in an eccentric manner.

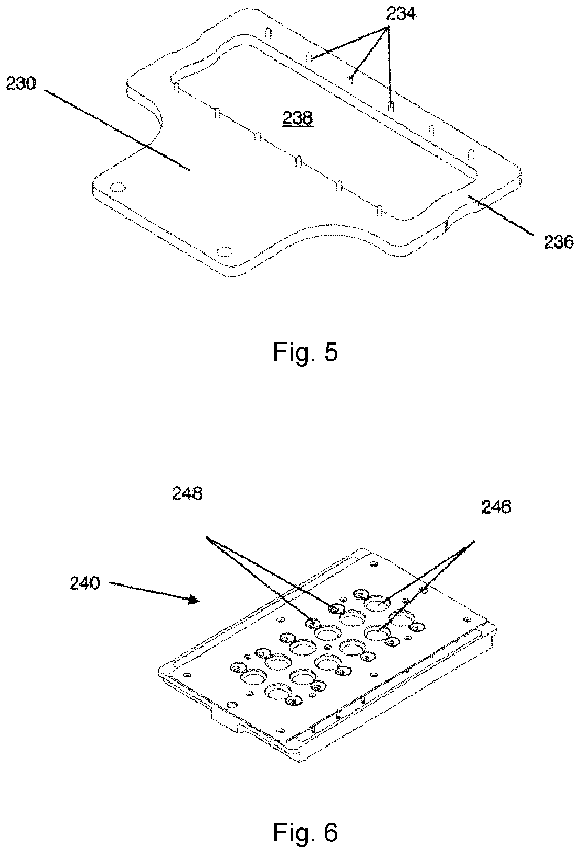

As best seen in Figure 5, the drive coupling 230 is a plate-like structure, having an array of twelve drive pins 234 projecting from the underside 236 thereof, each pin 234 corresponding to one of the vessel receiving locations 206. The drive pins 234 are arranged in two rows of six on either side of a cut-out 238 that extends through the centre of the structure for a purpose to be explained below.

A clamp plate 240 is removably connected to the cell culture module base 202, in a position overlying the vessel receiving station 204, by a pair of nuts 242 received on corresponding threaded posts 244 projecting from the upper surface 224 of the base. The nuts 242 preferably include a knurled exterior surface so as to be turnable without the use of tools. The clamp plate 240 is a generally rectangular, planar member having an array of relatively large circular apertures 246 arranged in two rows of six, in positions corresponding to the vessel receiving locations 206. See Figure 6. These apertures 246 are to accommodate the upstanding fluid transfer ports 406, with or without the associated caps 408 attached, of respective vessels 400 when received in the respective vessel receiving locations 206.

Adjacent to each of the relatively large apertures 246 is a smaller circular hole 248. A shaft is rotatably received in each of these smaller holes 248, each shaft having an off-axis hole sized and positioned to receive a respective one of the drive pins 234 of the drive coupling 230. The apertures 246 and the holes 248 all extend from top to bottom through the clamp plate 240. The underside of each shaft has a drive element for forming a drive connection with the drive input 424 of the stirrer 416 of a respective vessel 400, as described in more detail in EP A 2270129.

Twelve gas outlet ports (not shown) are located, in two rows of six, on the underside of the clamp plate 240 adjacent to the holes 248. The outlet ports are aligned with the gas input ports 412 of the respective vessels 400. Sealed connections are formed between the respective outlet ports and input ports 412 by virtue of an associated O-ring surrounding the outlet port. Twelve gas inlet ports (not shown) are located, in groups of three, at the corners of the underside of the clamp plate 240. These inlet ports are aligned with and form a sealed connection with the corresponding outlet ports 222 on the upper surface 224 of the base 202 when the clamp plate 240 is secured to the base 202. Each clamp plate outlet port is fluidically connected to a respective one of the clamp plate inlet ports. In this manner, each vessel 400 is connected to each of the gas supplies. The fluid connection may be made by means of plugged cross-drillings within the clamp plate 240. Alternatively, the fluid connection may be made via a flexible tube having a proximal end sealingly connected to a fluid terminal of the input port and a distal end sealingly connected to a fluid terminal of the output port. The arrangement and alignment of gas input and outlet ports is described in more detail in EP A 2270129.

A sensor assembly, described in more detail in EP A 2270129, is housed within the cavity defined by the frame 102 of the bed station 100. A sensor head is mounted on an X-Y positioning device for two-dimensional horizontal positioning within the cavity. The sensor head is thus able to be moved to and between respective sensing locations beneath the cell culture modules 200. Each sensor spot 426, 428 of each vessel 400 has an associated sensing location.

The sensing location will depend upon the type of sensor that is used. In the illustrated embodiment, in which each vessel 400 has both a pH sensor spot 426 and a DO sensor spot 428 on the bottom wall 403, the sensing locations for those spots are beneath the bottom wall 403. If the sensor spots were located on a side wall 404a, 404b, for example, the sensing locations might instead be adjacent to that side wall. The sensing head can include two sensors for respectively interrogating the pH sensor spot 426 and the DO sensor spot 428.

With reference again to Figure 1, the head 302 of the liquid handling station 300 includes a pipette tip handler that is selectively movable along the Z-axis and adapted to pick a pipette tip 306 and to dispense and/or aspirate fluids through the pipette tip. The head 302 also includes a capping device 308, also selectively movable along the Z-axis, for removing and replacing the vessel fluid transfer port caps 408. The head 302 further includes a suction cup 310 selectively movable along the Z-axis for removing and replacing the module lids 111.

Detailed discussion of use of the bioreactor processing system 10 to carry out an experimental run, in which bioreactor vessels 400 are loaded in respective vessel receiving locations 260 and the vessels are loaded with cell culture solution 407, is provided in EP A 2270129.

As an experiment run progresses, the cell culture solution 407 in each vessel 400 develops and has different requirements for optimum growth and production. During an experiment run, each vessel is individually monitored for pH and DO via the interrogation of the respective sensor spots 426, 428 by the sensor assembly.

The data stream from the sensor assembly is used as input to a computer-based controller (not shown) for feedback control of the input parameters for the individual vessels 400. For example, the data from the pH sensor can be used as an input to determine the quantity of CO₂ supplied to the individual vessels 400 with a view to keeping the pH within a predetermined profile.

Other parameters such as the stirring speed and the temperature of each culture station module 200 are also monitored and controlled as necessary. For example, the data from temperature sensors can be used as input to the heater(s) to ensure that the vessels 400 in the corresponding culture station module are maintained at a predetermined temperature profile.

Generally, an objective of an experiment run is to determine which set of process parameters provides optimum results. Accordingly, each vessel 400 in the unit 10 may be run with a slightly different set of parameters than the others. For example, different vessels 400 may be run with one or more of the following varied as compared to the other vessels: pH profile, CO₂ profile, DO profile, nutrients profile, temperature profile and stirring speed.

The best set of parameters may then be used as a reference point for further experiment runs.

Returning to Figure 2, the microscale bioreactor vessel 400 shown therein is similar to those used in the Ambr 15^{™} microbioreactor system, having a reserved culture volume (V_{rc}) for the cell culture solution placed in the vessel of 15 mL which is determined by the heights in the vessel of the connection joint of the sparge tube 140 to the gas input port 412 and the bottom of the drive input 424, and a total volume (Vₜₒₜ) inside the vessel of 23.4 mL, producing a reserved headspace volume of 8.4 mL. In use, the volume of cell culture solution placed in the vessel can be less than the reserved culture volume, the Ambr 15^{™} microbioreactor system, for example, allowing cell culture solution volumes of down to 10 mL.

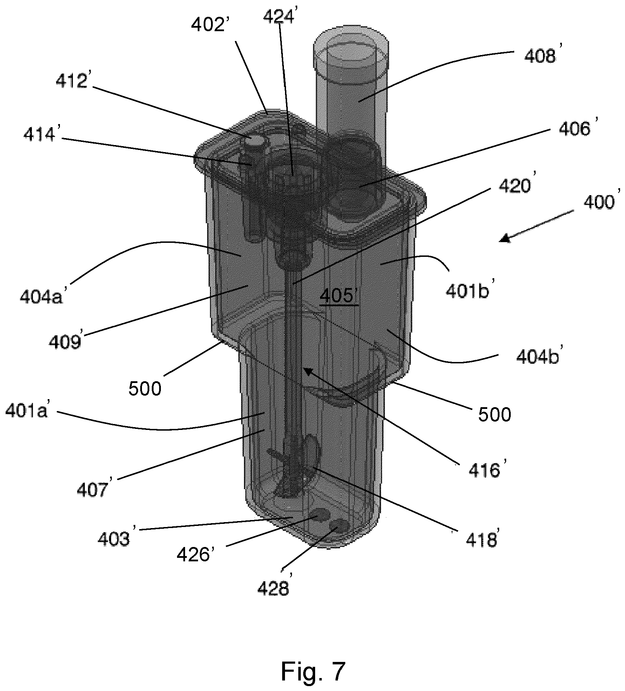

While the bioreactor vessels 400 of Figure 2 can be used for cell line selection and early process development, the 10 to 15 mL working volume range is an impediment to reducing the cost of experimentation. Accordingly, bioreactor vessels having a smaller working volume range can also be used with the bioreactor processing system 10. Figure 7 is a perspective view of such bioreactor vessel 400' for use in the bioreactor system. In Figure 7, features corresponding to those of Figure 2 have the same reference number but are distinguished from their Figure 2 counterparts by a prime.

Thus each vessel 400' comprises front, back, top, bottom and side walls 401a', 401b', 402', 403', 404a', 404b' defining a chamber 405' for receiving a cell culture solution 407' having a headspace 409' above. The top wall 402' includes a fluid transfer port 406', on which is removably attached a cap 408'. The vessel further includes a gas input port 412' in the top wall 402' of the vessel that extends into the headspace 409'. The gas input port 412' includes a filter 414'. However, unlike the vessel 400 of Figure 2, there is no sparge tube, and gas control is achieved by controlling the headspace gas only. By avoiding sparging, it is unnecessary to introduce and control small amounts of anti-foam agent that would otherwise be needed, small amounts of such agent being difficult to control and possibly having adverse effects on low volume cultures. Avoiding a sparger also eliminates the possibility of cell culture solution syphoning back up the sparge tube.

A stirrer 416' in the form of an impeller comprises blades 418' mounted at the base of a vertical shaft 420', and is rotatably mounted within the vessel 400'. The upper end of the shaft 420' includes a drive input 424'. A pH sensor spot 426' and a DO sensor spot 428' are disposed on the bottom wall 403', such that they are able to detect the pH and DO levels of the solution 407' and to be interrogated from the exterior of the vessel 400'.

Venting of the vessel chamber 405' is achieved via a labyrinthine path connecting the headspace 409' to atmosphere via the stirrer shaft drive input 424.' Alternatively, a separate vent port may be provided towards the top of the vessel 400'.

The side walls 404a' 404b' incorporate respective shoulders 500 which reduce the cross-sectional area of the chamber 405' below about mid-height. The reserved culture volume (V_{rc}) for cell culture solution 407' placed in the vessel 400' is determined by the height of these shoulders 500 and is 5.5 mL. The volume of solution cannot be allowed to exceed 5.5 mL, as the level of solution would then rise above the shoulders reducing the stirring effectiveness in the upper parts of the solution and thereby causing uneven process conditions throughout the solution. Thus the shoulders define a boundary between the reserved culture volume (V_{rc}) and the reserved headspace volume (Vᵣₕ). The total volume (Vₜₒₜ) of the vessel 400' is 17.3 mL, so that the reserved headspace volume (Vᵣₕ) for gas above the reserved culture volume is 11.8 mL, and Vᵣₕ/V_{rc} is 2.1.

The minimum culture volume (V_{minc}) for cell culture solution 407' in the vessel 400' is 2 mL and is determined by a need for the cell culture solution to fully cover the impeller blades 418'. At this depth of solution, there is also adequate coverage of the pH sensor spot 426' and the DO sensor spot 428'.

Thus the vessel 400' has a substantially smaller working volume for cell culture solution compared to the vessel 400, which can provide substantial cost savings in media and consumables. However, notwithstanding these differences in working volume, the vessel 400' interfaces to the bioreactor processing system 10 at its fluid transfer port 406', gas input port 412', drive input 424', pH sensor spot 426', and DO sensor spot 428' in the same way as the vessel 400. The pH sensor spot 426' and DO sensor spot 428' of the vessel 400' can be in different positions relative to the pH sensor spot 426 and DO sensor spot 428 of the vessel 400 as the sensor head of the sensor assembly can be easily controlled by the controller to move to these different positions. Moreover, the vessels 400' are removably receivable in the respective locations 206 of the receiving station 204 in the same way as the vessels 400. Thus the vessels 400' are interchangeable in the system 10 with the vessels 400 and closed-loop control of cell cultivation process parameters by the controller is performed similarly for both types of vessel. However, in order to more accurately position the vessels 400' in the X and Y directions at the locations 206 (which may be desirable if, for example, the sensor spots 426', 428' are reduced in size), an insert plate with apertures exactly matching the shapes of the top walls 402' of the vessels 400' may be loaded onto the receiving station 204 above the locations 206.

The configurations of both the vessel 400 and the vessel 400' adequately mimic those of larger scale vessels, which helps to ensure that cell culture solutions experience similar environmental conditions within these small-scale bioreactor as they would at larger scales. However, by providing vessels 400, 400' with different working volumes that can be used interchangeably in the same bioreactor processing system 10, the range of experimental conditions available to operators is substantially expanded without significantly increasing facility space and capital requirements. Thus in practice, an operator of the system 10 may have at their disposal two sets of bioreactor vessels for use in the system as they see fit: a first set being the vessels 400' with the smaller reserved culture volume (V_{rc}), and a second set being the vessels 400 with the larger reserved culture volume.

The vessels 400, 400' are both sterilizable by irradiation.

Various modifications can be made to the vessels 400, 400', particularly in the arrangement of the stirrer, without necessitating changes to the wider bioreactor processing system 10. For example, one option is to provide a pivot in the bottom wall 403, 403' to locate the lower end of the vertical shaft 420, 420'. This helps to ensure that the impeller is correctly positioned relative to the walls of the vessel, but increases a possibility of cell grinding at the interface between the pivot and the shaft. Another option is to replace the impeller completely with another stirring option such as a paddle that is moved in the cell culture solution with a circulating, e.g. orbital, motion rather than being spun on an axis like an impeller. Such paddles can accommodate a large range of fluid volumes in the bioreaction vessel and avoid the possibility of cell grinding.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventor does not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

## Claims

1. A bioreactor vessel (400') for performing cell cultivation, the vessel having a stirrer (418') within the vessel for stirring, in use, a cell culture solution (407') contained therewithin, and being configured for performance of closed-loop control of cell cultivation process parameters, wherein the vessel has a reserved culture volume (V_{rc}) for the cell culture solution and a reserved headspace volume (Vᵣₕ) for gas above the reserved culture volume, V_{rc} + Vᵣₕ being equal to the total volume (Vₜₒₜ) of the vessel, V_{rc} being less than 15 mL, and the ratio Vᵣₕ/V_{rc} being in the range from 0.5 to 3.

2. The bioreactor vessel according to claim 1, wherein the vessel has a gas inlet port (412') and a gas outlet path for controlling the composition of gas in the headspace (409') above the cell culture solution.

3. The bioreactor vessel according to claim 2, wherein the process parameters include flow rate of gas into the headspace through the gas inlet port.

4. The bioreactor vessel according to any one of the previous claims, wherein the reserved culture volume is 10 mL or less, preferably is 8 mL or less, and more preferably is 6 mL or less.

5. The bioreactor vessel according to any one of the previous claims, wherein the vessel has side walls (404a', 404b') which are configured to produce, at a predetermined height, a step change in the internal cross-sectional area of the vessel as measured perpendicularly to the height direction, the predetermined height defining a boundary between the reserved culture volume and the reserved headspace volume.

6. The bioreactor vessel according to any one of the previous claims, wherein the vessel is further configured to have a minimum culture volume for cell culture solution in the vessel of 2 mL or more.

7. The bioreactor vessel according to claim 6, wherein the minimum culture volume is determined by a requirement for the stirrer to enter the cell culture solution.

8. The bioreactor vessel according to any one of the previous claims, wherein the stirrer is an impeller.

9. The bioreactor vessel according to claim 8 as dependent on claim 7, wherein the minimum culture volume is determined by a requirement for the cell culture solution to fully cover the impeller.

10. A bioreactor system (10) including a cell culture module (200) comprising a base (202) having a receiving station (204) configured to removably receive and controllably interface to a plurality of bioreactor vessels (400') at respective vessel locations, the bioreactor system further including plural of the bioreactor vessels according to any one of the previous claims received at and interfaced to the respective vessel locations, and a controller to perform closed-loop control of the process parameters for each bioreactor vessel;
wherein the cell culture module further comprises:
a drive mechanism (226); and
a transmission (240) connectable to the drive mechanism for transmitting input motion from the drive mechanism into multiple rotary motion outputs, each output being removably connected to a stirrer (418') of a respective bioreactor vessel for rotation of that stirrer.

11. The bioreactor system according to claim 10, wherein the bioreactor vessels are as dependent on claim 2, and the transmission is a clamp plate removably connected to the base and containing respective gas lines for removably connecting to the gas inlet ports (412') of the bioreactor vessels.

12. The bioreactor system according to claim 10 or 11, wherein the bioreactor vessels are as dependent on claim 9 and the respective vessel locations have sensors (426', 428') for interfacing to the bioreactor vessels and measuring dissolved oxygen concentration in the culture solution and pH of the culture solution.

13. A kit of parts including a bioreactor system (10), and two sets of bioreactor vessels (400, 400') for performing cell cultivation;
wherein each vessel has a stirrer (418, 418') within that vessel for stirring, in use, a cell culture solution (407, 407') contained therewithin, is configured for performance of closed-loop control of cell cultivation process parameters, and has a reserved culture volume (V_{rc}) for the cell culture solution and a reserved headspace volume (Vᵣₕ) for gas above the reserved culture volume, V_{rc} + Vᵣₕ being equal to the total volume (Vₜₒₜ) of that vessel;
wherein the bioreactor vessels are identical to each other within each set, the bioreactor vessels (400') of a first one of the sets are bioreactor vessels according to any one of claims 1 to 9, and the reserved culture volume (V_{rc}) of the vessels of the first one of the sets is less than that of the vessels (400) of the second one of the sets;
wherein the bioreactor system includes a cell culture module (200) comprising a base (202) having a receiving station (204) configured to removably receive and controllably interface to the bioreactor vessels of each of the sets at respective vessel locations (206), and further includes a controller which, when the bioreactor vessels of one of the sets are received at and interfaced to the respective vessel locations, is configured to perform closed-loop control of the process parameters for each bioreactor vessel of that set; and
wherein the cell culture module further comprises:
a drive mechanism (226); and
a transmission (240) connectable to the drive mechanism for transmitting input motion from the drive mechanism into multiple rotary motion outputs, each output being removably connectable to a stirrer of a respective bioreactor vessel when received at and interfaced to a respective vessel location for rotation of that stirrer.

14. The kit according to claim 13, wherein the reserved culture volume (V_{rc}) of the vessels of the first one of the two sets is at least half that of the vessels of the second one of the two sets.

15. A method of performing cell cultivation including:
providing the bioreactor vessel of any one of claims 1 to 9;
introducing less than 10 mL of cell culture solution into the bioreactor vessel at a start of cell cultivation; and
performing closed-loop control of cell cultivation process parameters to cultivate cells within the introduced cell culture solution.
